# EUROPEAN PATENT SPECIFICATION

(11) **EP 0 741 143 B1**
(45) Date of publication and mention of the grant of the patent: **06.07.2005**
(21) Application number: 96106536.4
(22) Date of filing: 25.04.1996
(51) Int. Cl.: C07K 14/50, A61K 38/18

(54) **Fibroblast growth factor fragments**
Fibroblastenwachstumsfragmente
Fragments du facteur de croissance de fibroblastes

(30) Priority: 25.04.1995 JP 12594795
(43) Date of publication of application: 06.11.1996
(73) Proprietor: Ajinomoto Co., Inc., Tokyo (JP)
(72) Inventor: Oomura, Yutaka, c/o Insti. of Bio-Active Science, Yashiro-cho, Katoh-gun, Hyogo (JP)
(74) Representative: Hansen, Bernd, Dr. Dipl.-Chem.

(56) References cited:
- CHEMICAL ABSTRACTS, vol. 121, no. 15, 10 October 1994 Columbus, Ohio, US; abstract no. 170732x, page 143; XP002029324 & PHYSIOL. BEHAV., vol. 56, no. 2, 1994, pages 211-218, K SASAKI ET AL.: "Effects of fibroblast growth factors and related peptides on food intake in rats"
- CHEMICAL ABSTRACTS, vol. 124, no. 19, 6 May 1996 Columbus, Ohio, US; abstract no. 251144y, page 222; XP002029325 & EXP. NEUROL. , vol. 137, no. 2, 1996, pages 318-323, A LI ET AL.: "Fibroblast growth factor receptor-1 in the lateral hypothalamic area regulates food intake"

## Description

The present invention relates to a 1-29 amino acid residue peptide fragment of bovine acid fibroblast growth factor in which cysteine, an amino acid at the 16-position, is substituted with another amino acid and a pharmaceutically acceptable salt thereof. It also relates to the use of a pharmaceutical composition containing said peptide fragment as an effective ingredient.

### Background of the Invention

Fibroblast growth factor (FGF) is one of the peptidic cell growth factors, exhibits a growing action on cells such as fibroblast and endothelial cells of blood vessel and is a general term for a familty of several peptides mainly comprising basic FGF and acidic FGF. Acidic FGF (aFGF) is a protein with a molecular weight of about 16 kD comprising 140 amino acid residues, is widely distributed in various tissues in living organisms, is especially abundant in brain and retina, and has been known to exhibit various physiological actions necessary for maintaining the homeostasis of living body such as new growth of blood vessel due to cell growth action, cure of wounds, cure of ulcer and growth of undifferentiated osteogenic cells as well as improvement of memory and control of appetite.

As mentioned above, aFGF is a protein having many useful physiological activities. However, organic synthesis of a peptide having as many as 140 amino acid residues needs many steps of operation and is not advantageous in terms of economy and, therefore, its practical use is very difficult. Accordingly, gene recombination has been actually utilized but, in order to give a product of uniform quality, specialized techniques are required and, unlike in the case of organic synthesis, special facilities are necessary. In addition, FGF has a low organ specificity and has a possibility of conducting neovascular action to give cancer cells and, therefore, there is still a problem for its actual application as a pharmaceutical agent whereby manufacture of its derivatives and specific method for the administration have been investigated. Further, it is a protein having a molecular weight of 16 kD and, when compared with other peptides having small sizes, there are problems such as antigenicity and incorporating amount into brain and, consequently, there is still several points that have to be improved for its actual production as a pharmaceutical or the like meeting with a certain standard for use in an actual therapy.

Incidentally, there are some cases where, in achieving the activity of the physiologically active high-molecular substances, the use of total structure of the mature protein is not always necessary. Thus, it has been known that even a partial fragment often exhibits the same physiological activity as the parent protein provided that said fragment contains the active moiety of the mature protein. Saski et al., Chemical abstracts, vol. 121, no. 15, 10 October 1994, Columbus, Ohio, US, abstract no. 170732X, page 143; XP002029324 & Physiol. Behav., vol. 56, No. 2, 1994, pages 211-218, "Effects of fibroblast growth factors and related peptides on food intake in rats", describes that the acid fibroblast growth factor (aFGF) and aFGF fragments have a neurotrophic effect on neurons of the central nervous system, and act on the food intake of rats. However, a treatment of diseases such as Alzheimer Disease, dementia, or ischemic cerebral disease is not disclosed. In addition, when a part of amino acids participating in the active moiety of the peptide is substituted with other amino acids, it is possible to achieve reinforcement of biological activity, increase in stability, elongation of half-life, improvement in tissue specificity, improvement in incorporation into tissues, reduction of side effect, etc. of the peptide and its partial fragments. The present inventors have conducted investigations on partial peptides of aFGF which have various advantages such as the same physiological activity as aFGF has and the simple and easy synthesis whereupon they have found the peptide of the present invention.

### Detailed Description of the invention

An object of the present invention is to provide a 1-29 amino acid residue peptide fragment of bovine acid fibroblast growth factor
in which cysteine, an amino acid at the 16-position, is substituted with another amino acid which is useful as a pharmaceutical agent for improving cerebral function, etc.

The peptide fragment in accordance with the present invention is that of a 1-29 amino acid residue peptide fragment of bovine acid fibroblast growth factor in which cysteine, an amino acid at the 16-position, is substituted with another amino acid. Examples of the amino acid which is substituted for cysteine, an amino acid at 16-position of aFGF, are α-amino acids such as alanine, and glutamic acid. With regard to N-terminal, although aFGF in a living body is in a free type, that of an amide type can be easily prepared if a suitable synthetic method is applied and, therefore, the present invention includes such an amide type as well.

The following formula (II) shows an amino acid sequence of the peptide fragment of the present invention based on bovine aFGF.

5                   10                  15
H-Phe-Asn-Leu-Pro-Leu-Gly-Asn-Tyr-Lys-Lys-Pro-Lys-Leu-Leu-Tyr-
              20                  25
X-Ser-Asn-Gly-Gly-Thr-Phe-Leu-Arg-Ile-Leu-Pro-Asp-Gly-Y    (II)

In the formula, X is Ala, or Glu, and Y is OH or NH₂.

The peptide fragment of the present invention includes the pharmaceutically acceptable salts thereof such as acid addition salts with hydrochloric acid, sulfuric acid, nitric acid, hydrobromic acid, thiocyanic acid, boric acid, formic acid, acetic acid, haloacetic acid, propionic acid, glycolic acid, citric acid, tartaric acid, succinic acid, gluconic acid, lactic acid, malonic acid, fumaric acid, anthranilic acid, benzoic acid, cinnamic acid, p-toluenesulfonic acid, naphthalenesulfonic acid, sulfanilic acid and salts with alkali metal such as sodium and potasium, salts with alkaline-earth metal such as calcium and magnesium, or salts with other metals such as aluminum.

Those salts may be manufactured by known methods from the peptide fragment of the present invention in a free state or may be mutually converted among the salts. Amino acid residues of the peptide fragment of the present invention include any and all of their D-form, L-form and DL-form.

The peptide fragment of the present invention may be manufactured by a method which is common in peptide chemistry and any solid phase peptide synthesis, liquid phase peptide synthesis and gene technological means may be used. Each of the amino acids which constitute the peptide fragment of the present invention may be condensed one after another by the above-mentioned methods or it is also possible that partial peptides are previously synthesized and then they are condensed to manufacture the final peptide fragment of the present invention.

One example for a solid phase synthesis which is frequently used in the manufacture of peptides comprising scores of amino acid residues will be given as follows.

With regard to the solid phase peptide synthesis, a fundamental synthetic method is reported by Merrifield, et al. in J. Am. Chem. Soc., 85, page 2149 (1964) where an amino acid residue at C-terminal of the desired peptide is coupled with a suitable resin and then other amino acids are successively condensed therewith so that the peptide chain is elongated to the N-terminal. Thus, the amino acid residue corresponding to the C-terminal where the amino group is protected is bonded with a PAM [4-(hydroxymethyl)phenylacetamidomethyl) resin via a benzyl ester bond or with a BHA (benzhydrylamine) resin or an MBHA (methylbenzhydrylamine) resin via an amide bond whereby the initial stage of the synthetic reaction may be conducted. In case of separation and purification from each of the resins, the C-terminal may be obtained in a free type (where Y in the above formula is OH) and in an amide type (where Y in the above formula is NH₂) and the resin can be chosen depending upon the object. The resin to which one kind of amino acid residue is bonded as such is available on the market and it is convenient to use such a resin. Since the solid phase peptide synthesis utilizes the resin as a carrier, it is convenient when condensation and deblocking are successively carried out by changing the solvents and the process of washing the resin is quite simple as well.

Synthetic methods for peptides as well as condensing agents, protective groups, deblocking methods, solvents, reaction conditions, etc. are mentioned in detail in "Fundamentals and Experiments of Peptide Synthesis" by Nobuo Izumiya, et al. (published by Maruzen Co., Ltd.) and they may be suitably selected by conventional manner depending upon the type of the constituting amino acids of the aimed peptide, availability of the reagents, type of the synthetic devices, etc. In some reactions, protective groups for side chains are not necessary or, during the course of synthesis of the peptide fragment or in the final stage, they may be selectively or completely detached by conventional means such as a catalytic reduction or an acid decomposition using anhydrous hydrogen fluoride or trifluoroacetic acid. For example, Arg has amino acid even in the side chain and, therefore, the amino group participating in the condensation and that located in the guanidino group in the side chain are protected by a tert-butoxycarbonyl group (Boc) or by a p-toluenesulfonyl group (Tos), respectively so that the amino groups on the side chains are continuously protected until the final stage of the peptide synthesis (in which dicyclohexylcarbodiimide and trifluoroacetic acid are used as a condensing agent and an agent for detaching Boc, respectively) whereupon side reactions caused by the amino group of the guanidino group in the side chain can be prevented.

A brief explanation will be made by taking the case where the above-mentioned Boc is chosen as a protective group for the amino group participating in the condensation as an example. Thus, after the amino acid at the C-terminal of the aimed peptide is condensed with a resin, the group protected with Boc in said amino acid is detached by trifluoroacetic acid and then an amino acid (in which the amino group is protected by Boc) to be condensed is added followed by subjecting to a condensation whereupon a product in which dipeptide is bonded with the resin is prepared. When the same reaction is successively conducted, the peptide chain can be elongated accordingly and, at last, the aimed peptide can be liberated from the resin by treating with anhydrous hydrogen fluoride. In each step of the synthesis, the result of the condensation reaction can be confirmed by means of a ninhydrin reaction or the like. Thus, if the condensation takes place efficiently, free amino group is not present and, therefore, undetectable by a Kaiser test. Several types of peptide synthesizers where such a principle of peptide synthesis is applied are commercially available and it is possible to conduct an automatic peptide synthesis using such devices.

Usually, a high performance liquid chromatography (HPLC) and an amino acid analysis are combined for confirming whether the synthesized peptide has an aimed amino acid sequence. An example concerning the synthetic method of the peptide fragment of the present invention using a peptide synthesizer is given as hereunder.

### Brief Explanation of the Drawings

Fig. 1 shows the action of the peptide fragment of the present invention for improving the learning and the remembering in the passive avoidance learning test using the SAM-P/8.

Fig. 2 shows the action of the peptide fragment of the present invention for improving the space-recognizing ability reduced in the SAM-P/8 in the maze-learning test in water. The index is a diving time of a mouse until it escaped to the platform.

Fig. 3 shows the action of the peptide fragment of the present invention for improving the cerebral function in the maze-learning test in water by the same manner as in Fig. 2. This is a result where the staying time of the mouse in each of the divided four areas is used as an index.

### Examples

### Example 1.

A 1-29 amino acid residue peptide fragment of bovine aFGF in which Cys, an amino acid at the 16-position, was substituted with Ala was synthesized using a peptide synthesizer (Model 431A manufactured by Applied Biosystems Japan KK). PAM resin into which Boc-Gly was introduced was used as the resin and each of the amino acids protected by Boc was successively introduced whereupon the aimed peptide was synthesized. After removal of the protective groups and the resin, purification was conducted by means of a gel filtration and an HPLC. The resulting peptide was confirmed to be substantially pure as a result of the amino acid analysis and the HPLC.

Similarly, a 1-29 amino acid residue peptide fragment of bovine aFGF in which Cys, an amino acid at the 16-position, was substituted with Ala or Glu; was prepared by using the above-mentioned peptide synthesizer.

Senility-accelerated mice (SAM-P/8 strain) show physical senility symptoms at an early stage after birth and, at the same time, their abilities of learning and remembering are already lower in the third month from birth. Therefore, they are used as experimental models for dementia. The results of the investigation on the improving action of the peptide of the present invention on cerebral function using said senility-accelerated mice are given in detail as follows.

### (1) Passive avoidance learning test.

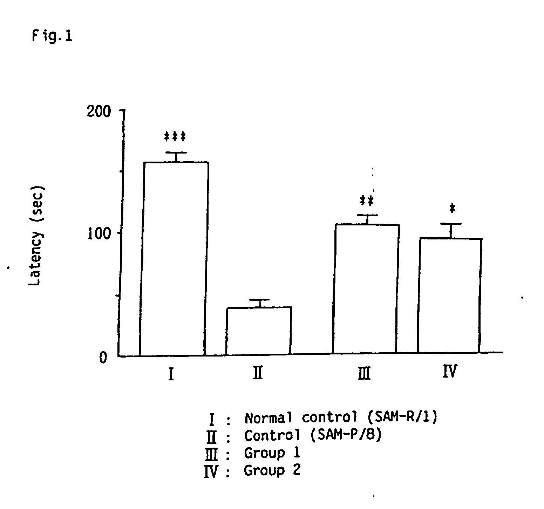

In a passive avoidance learning test, remembering ability of senility-accelerated mice of SAM-P/8 strain lowers since the third month from birth. The effect of the peptide of the present invention for improving said learning disability was investigated. Thus, a mouse was placed in front of a dark box and its ability of remembering the learning was measured using the time until it came into the box as an index. When electric current was turned on in the dark box after the mouse came into the box so that an electric shock was applied, the time until it came into the dark box was longer in a test trial after an acquisition trial in case the animal had a normal learning ability because it well remembered the electric shock. The test trial was conducted 24 hours after the acquisition trial. The peptide of the present invention was subcutaneously injected once a week since the third week after birth and its effect of improving the passive avoiding ability which was lowered in SAM-P/8 was investigated. Incidentally, a mouse (SAM-R/1) to which physiological saline solution was administered whereby aging proceeded normally and remembering ability did not lower was used as a normal control. An example of the results for SAM-P/8 on the sixth month after birth where the 1-29 amino acid residue peptide fragment of bovine aFGF in which Cys, an amino acid at 16-position, was substituted with Ala (150 µg/kg/day; group 1) and where the 1-15 amino acid residue fragment of bovine aFGF (80 µg/kg/day; group 2) were administered is given in Fig. 1 (t-test; *: p < 0.05; **: p < 0.01; ***: p < 0.001).

### (2) Water maze task (Morris' water maze task).

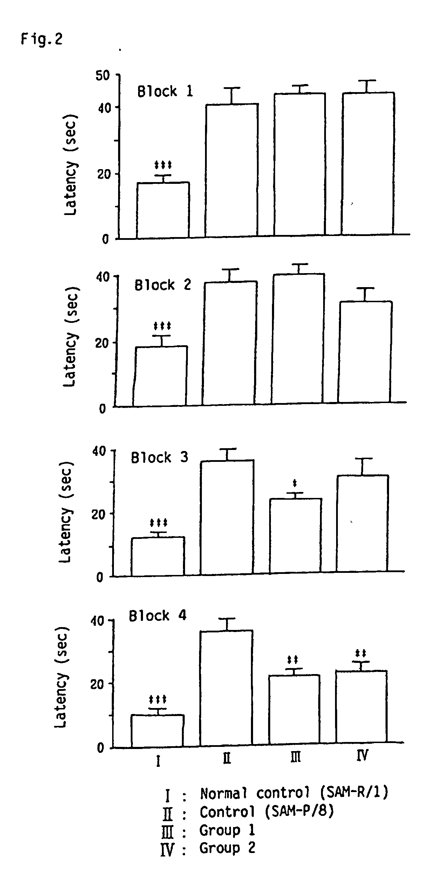

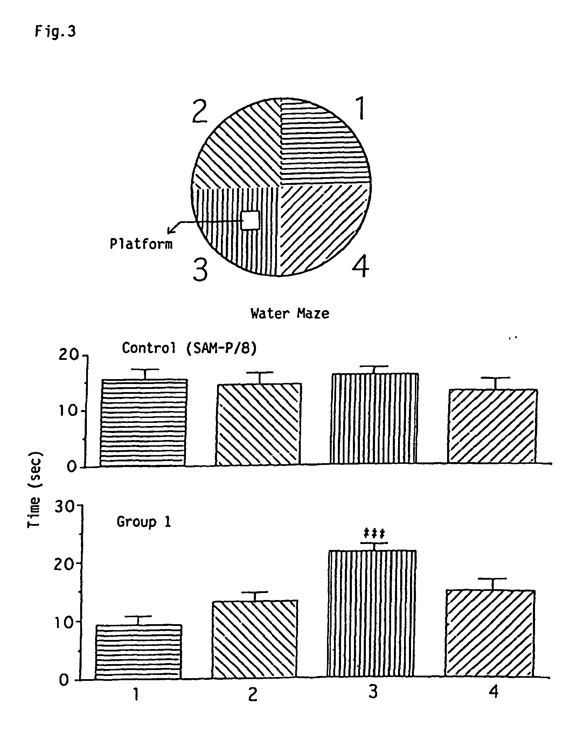

A platform was placed 1 cm under the water surface in a circular swimming pool for the experiment and then fine Styrofoam beads were uniformly sprinkled onto the water surface so that a mouse was unable to recognize the platform. Any hint to the mouse in the space around the device (such as persons in charge of the experiment, table, fluorescent light and machines for the experiment) was always kept constant during the experimental period. On the day of the experiment, the pool was divided into four equal areas (refer to Fig. 3), four starting points were randomly selected, the mouse was placed into water turning their head to the wall of the pool and its diving time until it escaped to the platform was measured. When the mouse climbed up to the platform, it was left as it was for 15 seconds. When the mouse did not climbed up even after 120 seconds, the trial was finished at that time and the mouse was placed on the platform, left as it was for 15 seconds the same as above and the diving time was recorded as 120 seconds. The case from one starting point was defined as one trial and four trials were done every ten minutes and defined as one block. Four blocks were carried out in total every other day. The peptide of the present invention was administered to the senility-accelerated mouce of an SAM-P/8 strain by the same manner as in the already-mentioned case of the passive avoiding learning test and an example of the results of the test of learning accelerating effect in ninth month from birth is given in Fig. 2. After the above-mentioned four blocks were conducted, the platform was removed, the mouse was left to swim for a certain period and the time for swimming in each of the four areas was measured. When the mouse recognized and remembered the location of the platform, it stayed for a long time at the place where the platform was. Therefore, the learning and remembering abilities could be determined measuring the staying time in each of the four areas. An example of the results is given in Fig. 3.

Additionally, the effects of the peptides of the present invention on neuronal death in the CAI subfields of the hippocampus are given as follows.

The peptide fragment of the present invention, the 1-29 amino acid residue peptide fragment of bovine aFGF in which Cys, an amino acid at the 16-position, was substituted with Ala or the 1-29 amino acid residue peptide fragment of bovine aFGF in which Cys, an amino acid at the 16-position, was substituted with Ser, was administered by continuous intracerebroventricular infusion (850 µg/ml, respectively) into the lateral cerebral ventricles of Wister-strain rat (250-300 g, 4 rats/group) and their common carotid arteries were occluded for 15 minutes to induce ischemic insult. 5 days after ischemia, the rats were deeply anesthetized with sodium pentobarbital and transcardially perfused. Then, the brains were frozen and cut coronally in 20 µm sections by microtomy, and stained by Nissl bodies. The sections were examined by histological analysis. As a result of the analysis, the protecting effect of the peptide of the present invention on neuronal death in the CAI subfields of the hippocampus was observed (Ala-substituted peptide, 3/4 rats; Ser-substituted peptide, 4/4 rats).

As shown in Fig. 1, when the peptide fragment of the present invention is administered to senility-accelerated mice of an SAM-P/8 strain exhibiting disabled learning and remembering with aging in the passive avoidance learning test, an action of significantly improving the reduced acquiring and maintaining abilities for remembering and learning in the SAM-P/8 strain was noted. The space-recognizing ability of the SAM-P/8 strain was also reduced in the maze-learning test in water (cf. Figs. 2 and 3) but the peptide fragment of the present invention showed an action of a significant improvement for that. It was also observed that the peptide fragment of the present invention have the protecting effects against the death of cerebral neurons caused by ischemia.

It is apparent from the above-mentioned results of pharmacological tests that the peptide fragments of the present invention exhibit excellent actions of improving the cerebral functions and protecting the cerebral neurons. Consequently, the peptide fragment of the present invention is highly useful in a therapy of diseases resulting in a reduced cerebral functions such as diseases caused by degeneration of brain (e.g. Alzheimer disease, dementia) ischemic cerebral diseases (e.g. cerebral infarction and cerebral thrombosis) and also in an improvement of various symptoms accompanied thereby such as hypomnesia, aphasia, disturbance of consciousness, depressive state, indifference, delusion and confusion.

The peptide fragment of the present invention can be made into a pharmaceutical agent by combining with a suitable pharmaceutical carrier or diluent and can be made into pharmaceutical preparations by any of the common methods. In the prescription, the substance of the present invention may be used in a form of a pharmaceutically acceptable salt thereof and the peptide fragment of the present invention may be used either solely or jointly in a suitable manner or may be compounded with other pharmaceutically active ingredients. Since the substance of the present invention is a peptide, it is common that the substance is usually prepared into injection solution. Thus, the substance may be prepared in a form of solution or suspension in aqueous or nonaqueous solvent such as distilled water for injection, physiological saline solution, Ringer solution, plant oil, synthetic fatty acid glycerides, esters of higher fatty acids and propylene glycol and, in addition, frequently-used additives for pharmaceuticals such as stabilizers, buffers, suspending agents, isotonic agents, pH adjusting agents, antiseptics and preservatives may be suitably added thereto as well. It is also possible that, depending upon the type of the disease, other pharmaceutical preparations which are optimum for the therapy of said disease such as special preparations whereby the substance is not decomposed even by oral administration may be prepared to prescribe into the preparation for oral administration.

## Claims

1. Use of a 1-29 amino acid residue peptide fragment of bovine acidic fibroblast growth factor, in which a cystein, an amino acid at the 16-position, is substituted with alanine or glutamic acid, wherein the peptide fragment is 
H·Phe·Asn·Leu·Pro·Leu·Gly·Asn·Tyr·Lys·Lys·Pro·Lys·Leu·
Leu·Tyr·X·Ser·Asn·Gly·Gly·Thr·Phe·Leu·Arg·Ile·Leu·Pro·
Asp·Gly·Y,
 wherein X represents Ala or Glu and Y represents OH or NH₂, or a pharmaceutically acceptable salt thereof, for the preparation of a medicament for treating Alzheimer disease, dementia or ischemic cerebral disease.

## Patentansprüche

1. Verwendung eines 1-29 Aminosäurerest-Peptidfragments von bovinem saurem Fibroblastenwachstumsfaktor, worin ein Cystein, eine Aminosäure an der 16-Position, mit Alanin oder Glutaminsäure substituiert ist, wobei das Peptidfragment das folgende ist: 
H·Phe·Asn·Leu·Pro·Leu·Gly·Asn·Tyr·Lys·Lys·Pro·Lys·Leu·
Leu·Tyr·X·Ser·Asn·Gly·Gly·Thr·Phe·Leu·Arg·Ile·Leu·Pro·
Asp·Gly·Y
 worin X Ala oder Glu bedeutet und Y bedeutet OH oder NH₂, oder eines pharmazeutisch akzeptablen Salzes davon für die Herstellung eines Medikaments zur Behandlung der Alzheimerschen Erkrankung, der Demenz oder der ischämischen zerebralen Erkrankung.

## Revendications

1. Utilisation d'un fragment peptide de résidu d'acide aminé 1-29 du facteur de croissance de fibroblastes acides bovins, dans laquelle une cystéine, un acide aminé en position 16, est substitué par une alanine ou un acide glutamique, dans laquelle le fragment peptide est 
H·Phe·Asn·Leu·Pro·Leu·Gly·Asn·Tyr·Lys·Lys·Pro·Lys·Leu·
Leu·Tyr·X·Ser·Asn·Gly·Gly·Thr·Phe·Leu·Arg·Ile·Leu·Pro·
Asp·Gly·Y,
 dans lequel X représente Ala ou Glu et Y représente OH ou NH₂ , ou un sel pharmaceutiquement acceptable de celui-ci, pour la préparation d'un médicament destiné au traitement de la maladie d'Alzheimer, de la démence ou d'une maladie d'ischémie cérébrale.
